# EUROPEAN PATENT APPLICATION

(11) **EP 1 977 692 A2**
(43) Date of publication of application: **08.10.2008**
(21) Application number: 08006511.3
(22) Date of filing: 31.03.2008
(51) Int. Cl.: A61B 6/00, A61B 6/04, A61B 8/08

(54) **Ultrasonic diagnosis apparatus, breast imaging system, and breast imaging method**

(30) Priority: 05.04.2007 JP 2007099691
(71) Applicant: Kabushiki Kaisha Toshiba, Minato-ku, Tokyo 105-8001 (JP); Toshiba Medical Systems Corporation, Otawara-shi, Tochigi-ken 324-8550 (JP)
(72) Inventor: Okamura, Yoko, Otawara-shi Tochigi 324-8550 (JP); Kamiyama, Naohisa, Otawara-shi Tochigi 324-8550 (JP); Sugiyama, Atsuko, Otawara-shi Tochigi 324-8550 (JP); Yamagata, Hitoshi, Otawara-shi Tochigi 324-8550 (JP); Nishiki, Masayuki, Otawara-shi Tochigi 324-8550 (JP); Kuratomi, Naoko, Otawara-shi Tochigi 324-8550 (JP); Seo, Yasutsugu, Otawara-shi Tochigi 324-8550 (JP)
(74) Representative: Kramer - Barske - Schmidtchen

(57) **Abstract**

A compression paddle of a mammographic diagnosis apparatus (1) is provided with an opening portion (350) which allows an ultrasonic probe (62) to be fitted therein and slide. This apparatus performs ultrasonic scanning on a breast compressed/fixed to the compression paddle, and reconstructs a compressed breast image by using the obtained ultrasonic image as a virtual image, thereby acquiring an ultrasonic image and volume data of the compressed/fixed breast.

## Description

The present invention relates to a system used for combined breast imaging of X-ray imaging and ultrasonic imaging, an ultrasonic diagnosis apparatus used for the integrated imaging, and a breast imaging system.

An ultrasonic diagnosis technique can display, in real time, how a heart beats or a fetus moves, with simple operation of bringing an ultrasonic probe into contact with the body surface. In addition, this technique offers a high level of safety, and hence can be repeatedly used for examination. Furthermore, the system size is smaller than those of other diagnosis apparatuses such as X-ray, CT, and MRI apparatuses. Therefore, this apparatus allows easy examination upon being moved to a bed side. That is, the apparatus is a convenient diagnosis technique. Ultrasonic diagnosis apparatuses used in such ultrasonic diagnosis vary depending on the types of functions which they have. Some compact apparatuses which can be carried with one hand have been developed. Ultrasonic diagnosis is free from the influence of radiation exposure such as X-ray exposure, and hence can be used in obstetric treatment, treatment at home, and the like.

This ultrasonic diagnosis apparatus is frequently used for examination of breast cancer like mammography (MMG) using a mammographic diagnosis apparatus. Examination using MMG (MMG examination) is excellent in detection performance for micro calcification. On the other hand, it is said that ultrasonic examination is superior in extraction of biological tissue structures and tumors. MMG examination and ultrasonic examination have their own merits and demerits and play complementary roles in breast cancer examination. Therefore, positionally associating images (mammographic images) obtained by MMG with ultrasonic images allows to expect an improvement in diagnosis accuracy.

However, the following problems arise in integrated breast imaging of conventional X-ray imaging and ultrasonic imaging.

General MMG examination requires breast compression. In contrast, ultrasonic examination performs ultrasonic scanning on breasts in a non-compressed state. For this reason, the shapes of breasts change at the time of image acquisition, and hence it is difficult to accurately positionally associate mammographic images with ultrasonic images.

In order to positionally associate mammographic images with ultrasonic images, there have been proposed a method of performing ultrasonic scanning over a compression paddle in a mammographic diagnosis apparatus, a method of performing ultrasonic examination between the compression paddles, and the like. According to such a technique, however, in order to acquire an ultrasonic image of an entire breast, it is necessary to compress the breast with compression paddles having a plurality of holes for the insertion of an ultrasonic probe and acquire an ultrasonic image at the position of each hole while sequentially shifting the ultrasonic probe. This operation requires long time to acquire an ultrasonic image. In addition, in some cases, compressing breasts with compression paddles having a plurality of holes causes pain in subjects to be examined. This imposes heavy burden on the subjects. In addition, compressing a breast with compression paddles having a plurality of holes may lead to a reduction in compression strength.

The present invention has been made in consideration of the above situation, and has as its object to provide an ultrasonic diagnosis apparatus, breast imaging system, and breast imaging method which can accurately positionally associate mammographic images with ultrasonic images while shortening the time required to acquire ultrasonic images and reducing pain accompanying compression as compared with the prior art.

According to an aspect of the present invention, there is provided a breast imaging system for acquiring an X-ray image and ultrasonic image of a breast, the system comprising an X-ray application unit which applies X-rays, an X-ray detection unit which detects X-rays striking a detection surface, a compression unit which has an opening portion in which an ultrasonic probe is to be placed and compresses the breast by sandwiching the breast between a first paddle and a second paddle, an X-ray image generating unit which generates an X-ray image on the basis of X-rays detected by the X-ray detection unit, an ultrasonic probe which is placed in the opening portion, and generates an echo signal by transmitting an ultrasonic wave to the compressed breast on the basis of a supplied driving signal, and receiving a reflected wave from the breast, a transmission unit which supplies the driving signal to the ultrasonic probe, and an image generating unit which generates an ultrasonic image of the compressed breast on the basis of the echo signal and information concerning a propagation path of an ultrasonic wave including reflection of an ultrasonic wave by the compression unit.

According to another aspect of the present invention, there is provided an ultrasonic diagnosis apparatus comprising an ultrasonic probe which generates an echo signal by transmitting an ultrasonic wave to a breast compressed by the compression paddle on the basis of a supplied driving signal, and receiving a reflected wave from the breast, a transmission unit which supplies the driving signal to the ultrasonic probe, and an image generating unit which generates an ultrasonic image of the compressed breast on the basis of the echo signal and information concerning a propagation path of an ultrasonic wave including reflection of an ultrasonic wave by the compression unit.

According to yet another aspect of the present invention, there is provided a breast imaging method of acquiring an X-ray image and ultrasonic image of a breast, the method comprising applying X-rays to a breast compressed by a compression unit having an opening portion in which an ultrasonic probe is to be placed, a first paddle, and a second paddle, detecting X-rays transmitted through the breast, generating an X-ray image on the basis of the detected X-rays, placing the ultrasonic probe in the opening portion and transmitting an ultrasonic wave to the compressed breast on the basis of a supplied driving signal, generating an echo signal by receiving a reflected wave from the breast, and generating an ultrasonic image of the compressed breast on the basis of the echo signal and information concerning a propagation path of an ultrasonic wave including reflection of an ultrasonic wave by the compression unit.

According to yet another aspect of the present invention, there is provided a breast imaging method comprising transmitting an ultrasonic wave to a breast compressed by a compression unit on the basis of a supplied driving signal, generating an echo signal by receiving a reflected wave from the breast, and generating an ultrasonic image of the compressed breast on the basis of the echo signal and information concerning a propagation path of an ultrasonic wave including reflection of an ultrasonic wave by the compression unit.

The invention can be more fully understood from the following detailed description when taken in conjunction with the accompanying drawings, in which:
FIG. 1 is a perspective view showing the outer appearance of a breast imaging system 1 according to an embodiment;
FIG. 2 is a block diagram showing the arrangement of a mammographic diagnosis apparatus 2;
FIG. 3 is a perspective view showing an example of the arrangement of a compression unit 35;
FIG. 4 is a view showing how a breast is compressed by the compression unit 35;
FIG. 5 is a perspective view showing another example of the compression unit 35;
FIG. 6 is a block diagram showing the arrangement of an ultrasonic diagnosis apparatus 6;
FIGS. 7A and 7B are views for explaining a compressed breast image reconstruction function;
FIG. 8 is a view for explaining how a compressed breast image is positionally associated with a mammographic image;
FIG. 9 is a flowchart showing each processing sequence executed in image acquisition using the breast imaging system 1;
FIG. 10 is a perspective view showing the arrangement of a compression unit 35 according to the second embodiment; and
FIG. 11 is a view for explaining a compressed breast image reconstruction function according to the fourth embodiment.

The first and second embodiments of the present invention will be described below with reference to the views of the accompanying drawing. Note that the same reference numerals denote constituent elements having substantially the same functions and arrangements in the following description, and a repetitive description will be made only when required.

### (First Embodiment)

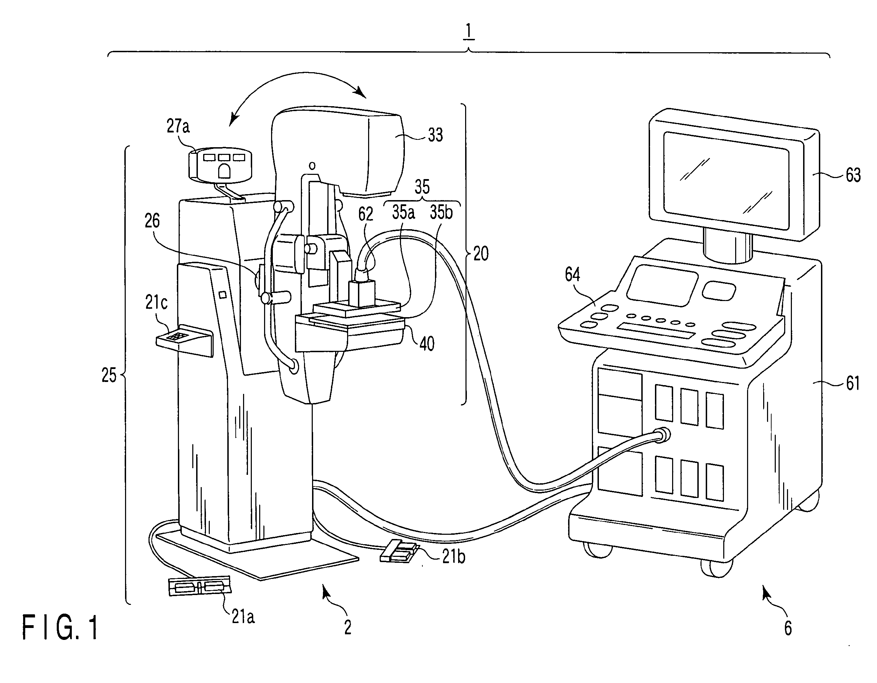

FIG. 1 is a perspective view showing the outer appearance of a breast imaging system 1 according to this embodiment. As shown in FIG. 1, the breast imaging system 1 comprises a mammographic diagnosis apparatus 2 and an ultrasonic diagnosis apparatus 6. The arrangement of each apparatus will be described below.

### (Mammographic Diagnosis Apparatus)

As shown in FIG. 1, the mammographic diagnosis apparatus 2 comprises an arm portion 20 and a support portion 25. The arm portion 20 is provided with an X-ray source device 33 and flat panel detector 40 which are placed to face each other, a compression unit 35 comprising upper and lower paddles 35a and 35b, and the like. The support portion 25 is provided with a display panel 27a, foot pedals 21a and 21b, a touch panel 21c, and the like. The arm portion 20 can move vertically with respect to the support portion 25 and rotate about a shaft 26. When the arm portion 20 is kept in a predetermined posture and a breast is compressed by the upper paddle 35a and the lower paddle 35b provided on the detection surface of the flat panel detector 40, images can be acquired in arbitrary directions such as the cephalocaudal direction, medio-lateral direction, and medio-lateral oblique direction.

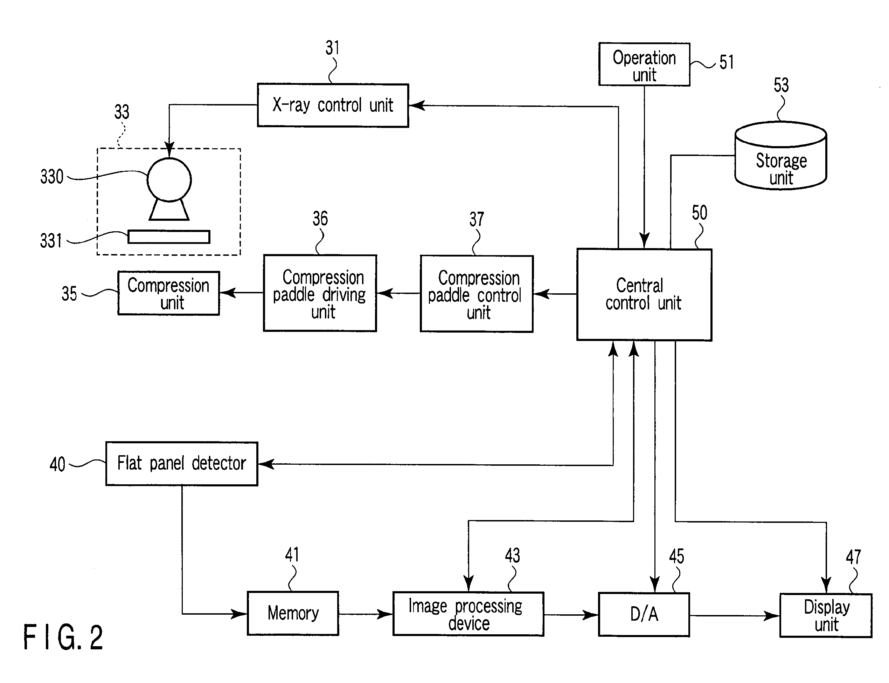

FIG. 2 is a block diagram showing the arrangement of the mammographic diagnosis apparatus 2. As shown in FIG. 2, the mammographic diagnosis apparatus 2 comprises an X-ray control unit 31, the X-ray source device 33, the compression unit 35, a compression paddle driving unit 36, a compression paddle control unit 37, the flat panel detector 40, a memory 41, an image processing device 43, a D/A converter 45, a display unit 47, a central control unit 50, an operation unit 51, and a storage unit 53.

The X-ray control unit 31 controls the X-ray source device 33 to apply X-rays with a predetermined intensity at a predetermined rate in accordance with an instruction from the central control unit 50.

The X-ray source device 33 includes an X-ray tube 330 and an X-ray irradiation field limiter 331. The X-ray tube 330 is a vacuum tube which generates X-rays. The X-ray tube 330 generates X-rays by accelerating electrons using a high voltage from a high voltage generator and bombarding the electrons on a target. The X-ray irradiation field limiter 331 forms the X-rays applied from the X-ray tube 330 into a predetermined shape.

The compression unit 35 comprises the upper and lower paddles 35a and 35b, and is provided between the X-ray tube 330 and the flat panel detector 40. A breast to be examined is fixed in a flat state at the time of imaging by being sandwiched and compressed between the upper and lower paddles 35a and 35b. The upper and lower paddles 35a and 35b are made of a material having both radiolucency and ultrasonic reflectivity (e.g., a sufficiently polished acrylic plate). Compressing and fixing (positioning) a breast between the upper and lower paddles can reduce scattered X-rays from the subject and the overlap of mammary gland tissues, thereby, for example, improving image contrast, preventing the occurrence of noise due to body movement, and reducing the irradiation dose.

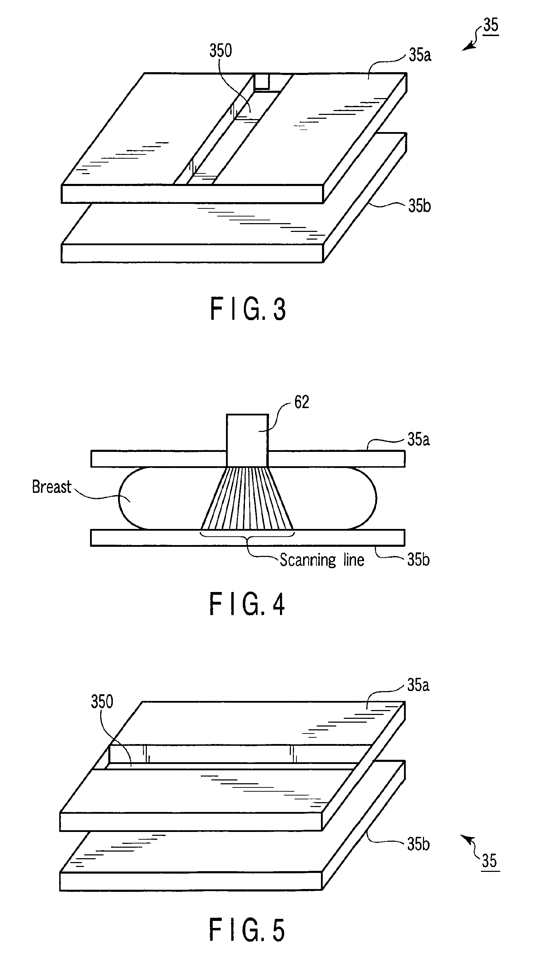

Note that the lower paddle 35b of the compression unit 35 need not be integrated with the flat panel detector 40. In addition, as shown in FIG. 3, an opening portion 350 in which a one-dimensional ultrasonic probe is slidably placed is formed in the upper paddle 35a of the compression unit 35. As shown in FIG. 4, an ultrasonic probe is placed in the opening portion 350 while the breast is compressed/fixed between the upper and lower paddles 35a and 35b. Ultrasonic scanning is then performed while the probe is slid. This can obtain an ultrasonic image in the same state as at the time of mammography.

FIG. 3 shows an example of the shape of the opening portion 350. That is, the opening portion 350 can have any shape as long as volume scanning can be performed by sliding the one-dimensional ultrasonic probe while the breast is compressed/fixed. As shown in FIG. 5, therefore, even if the opening portion is formed in a direction perpendicular to that in FIG. 3, the same function and effect can be obtained. In addition, in order to prevent the ultrasonic probe from moving vertically at the time of volume scanning, it is preferable to provide the opening portion 350 with a thin film which is acoustically transparent and has compression force large enough to prevent the breast from greatly protruding.

This embodiment uses the arrangement comprising the upper and lower paddles 35a and 35b. However, the present invention is not limited to this. For example, the lower paddle 35b need not be provided if the detection surface of the flat panel detector 40 has high ultrasonic reflectivity, compression durability, and the like and can be used as the lower paddle 35b.

The compression paddle driving unit 36 drives the compression unit 35 under the control of the compression paddle control unit 37.

The compression paddle control unit 37 controls the compression paddle driving unit 36 to perform positioning using the compression unit 35 in accordance with an instruction from the central control unit 50. The compression paddle control unit 37 measures the compression thickness of the compressed/fixed breast (i.e., the distance between the upper and lower paddles 35a and 35b) and sends out the resultant data to the central control unit 50.

The flat panel detector 40 includes a scintillator and a photodiode array. The flat panel detector 40 generates electrons/holes by causing X-rays transmitted through a subject to strike a photoelectric film, stores the electrons/holes in a semiconductor switch, and reads them as an electrical signal, thereby converting the X-rays into the electrical signal and detecting it. Note that this detector may use, as a conversion scheme, a direct conversion scheme of converting X-rays into an electrical signal or an indirect conversion scheme of converting X-rays into an electrical signal through light.

The memory 41 temporarily stores a digital signal supplied from the flat panel detector 40.

The image processing device 43 reads out a digital signal from the memory 41 for each frame, and performs predetermined image processing such as subtraction processing as needed.

The D/A converter 45 converts the digital signal sequence of the image data input from the image processing device 43 into an analog signal sequence.

The display unit 47 includes the display panel 27a displaying the operation state of the apparatus in addition to a CRT, plasma display, liquid crystal display, or the like which displays an X-ray diagnostic image obtained from a signal received from the image processing device 43.

The central control unit 50 is a central processing device which performs control concerning the acquisition of image data, image processing for the acquired image data, and control concerning image playback processing and the like. At the time of acquisition of a compressed breast image (to be described later), the central control unit 50 transmits the compression thickness of the compressed/fixed breast, the position of an ultrasonic probe 62, provided on the upper paddle 35a, on the detection surface of the flat panel detector 40, and the like to the ultrasonic diagnosis apparatus 6 as needed. Note that the position of the ultrasonic probe 62, provided on the upper paddle 35a, on the detection surface of the flat panel detector 40 can be acquired by providing a sensor or the like, which detects the position of the ultrasonic probe 62, in the opening portion 350 of the upper paddle 35a.

The operation unit 51 is an input device including a keyboard, various switches, a mouse, the foot pedals 21a and 21b, the touch panel 21c, and the like. The operation unit 51 is used to, for example, input patient information (a patient ID, examination region, examination purpose, and the like), vertically move the compression unit 35, issue an imaging instruction, select a pulse rate, select an image and the like.

The storage unit 53 stores image data or the like before or after image processing by the image processing device 43. The storage unit 53 stores a dedicated program for implementing a digital automatic exposure control function to be described later.

### (Ultrasonic Diagnosis Apparatus)

As shown in FIG. 1, the ultrasonic diagnosis apparatus 6 comprises an apparatus body 61, an ultrasonic probe 62, a monitor 63, and an input device 64.

The ultrasonic probe 62 includes a plurality of piezoelectric transducers which generate ultrasonic waves on the basis of driving signals from the apparatus body 61 and convert reflected waves from a subject to be examined into electrical signals, a matching layer provided for the piezoelectric transducers, a backing member which prevents ultrasonic waves from propagating backward from the piezoelectric transducers, and the like. When an ultrasonic wave is transmitted from the ultrasonic probe 62 to a subject P to be examined, the transmitted ultrasonic wave is sequentially reflected by a discontinuity surface of acoustic impedance of internal body tissue, and is received as an echo signal by the ultrasonic probe 62. The amplitude of this echo signal depends on an acoustic impedance difference on the discontinuity surface by which the echo signal is reflected. The echo produced when an ultrasonic pulse is reflected by the surface of a moving blood flow, cardiac wall, or the like is subjected to a frequency shift depending on the velocity component of the moving body in the ultrasonic transmission direction due to a Doppler effect.

The input device 64 is connected to the apparatus body 61. The input device 64 has various types of switches, buttons, a trackball, a mouse, a keyboard, and the like which are used to input, to the apparatus body 61, various types of instructions and conditions, an instruction to set a region of interest (ROI), various types of image quality condition setting instructions, and the like from an operator. When, for example, the operator operates the end button or FREEZE button of the input device 64, the transmission/reception of ultrasonic waves is terminated, and the ultrasonic diagnosis apparatus is set in a pause state.

The monitor 63 displays morphological information and blood flow information in the living body as images on the basis of video signals from the apparatus body 61.

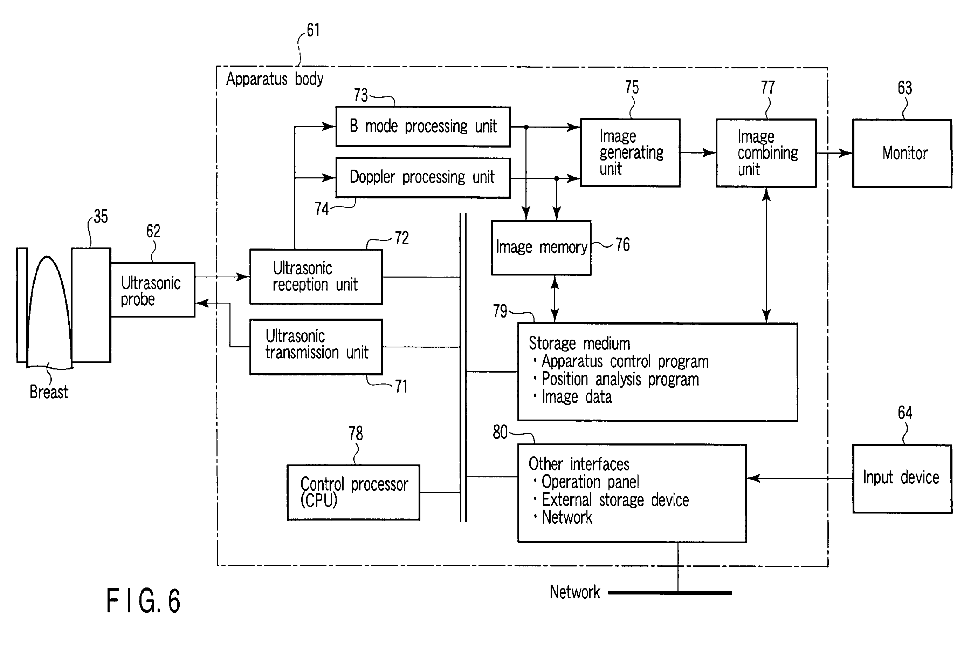

FIG. 6 is a block diagram showing the arrangement of the ultrasonic diagnosis apparatus 6. As shown in FIG. 6, the apparatus body 61 of the ultrasonic diagnosis apparatus 6 includes an ultrasonic transmission unit 71, an ultrasonic reception unit 72, a B mode processing unit 73, a Doppler processing unit 74, an image generating unit 75, an image memory 76, an image combining unit 77, a control processor (CPU) 78, an internal storage unit 79, and an interface unit 80. The function of each constituent element will be described below.

The ultrasonic transmission unit 71 has a trigger generating circuit, delay circuit, pulser circuit, and the like (none are shown). The pulser circuit repeatedly generates rate pulses for the formation of transmission ultrasonic waves at a predetermined rate frequency fr Hz (period: 1/fr sec). The delay circuit gives each rate pulse the delay time required to focus an ultrasonic wave into a beam for each channel and determine a transmission directivity. The trigger generating circuit applies a driving pulse to the ultrasonic probe 62 at the timing based on this rate pulse.

The ultrasonic transmission unit 71 has a function of instantly changing a transmission frequency, a transmission driving voltage, or the like to execute a predetermined scan sequence in accordance with an instruction from the control processor 78. A change in transmission driving voltage, in particular, is implemented by a linear amplifier type transmission circuit capable of instantly switching the value of the voltage or a mechanism of electrically switching a plurality of power supply units.

The ultrasonic reception unit 72 has an amplifier circuit, A/D converter, adder, and the like (none are shown). The amplifier circuit amplifies echo signals received via the ultrasonic probe 62 on a channel basis. The A/D converter gives each amplified echo signal the delay time required to determine a reception directivity. The adder then performs addition processing. With this addition, the reflection component of the echo signal from the direction corresponding to the reception directivity is enhanced, and a synthetic beam for ultrasonic transmission/reception is formed in accordance with the reception directivity and transmission directivity.

The B mode processing unit 73 receives the echo signal from the ultrasonic reception unit 72, and performs logarithmic amplification, envelope detection processing, and the like, thereby generating data whose signal strength is represented by a brightness level.

The Doppler processing unit 74 frequency-analyzes velocity information from the echo signal received from the ultrasonic reception unit 72 to extract a blood flow, tissue, and contrast medium echo component by the Doppler effect, and obtains blood flow information such as an average velocity, variance, and power at multiple points. The obtained blood flow information is sent to the image generating unit 75 to be color-displayed as an average velocity image, a variance image, a power image, and a composite image thereof on the monitor 63.

The image generating unit 75 converts a signal sequence (an ultrasonic scanning line signal sequence) from the B mode processing unit 73 into a scanning line signal sequence in a general video format typified by a TV format to generate an ultrasonic diagnostic image as a display image. At this time, applying various types of image filters such as an edge emphasis filter, time filter, and space filter to the signal can provide image quality according to user preference. Note that data before it is input to the image generating unit 75 is sometimes called "raw data".

The image memory 76 is, for example, a memory which stores ultrasonic images corresponding to a plurality of frames immediately before freezing. Performing continuous display (cine display) of images stored in the image memory 76 can display a moving ultrasonic image. Note, however, that this image includes a virtual image.

The image combining unit 77 combines the image received from the image generating unit 75 with character information of various types of parameters, scale marks, and the like, and outputs the resultant signal as a video signal to the monitor 63. The image combining unit 77 also executes, under the control of the control processor 78, a compressed breast image reconstruction function (to be described later) of reconstructing an ultrasonic image of an actual breast (i.e., an ultrasonic image (compressed breast image) of the breast compressed/fixed by the compression unit 35) by using an ultrasonic image as a virtual image obtained by using ultrasonic waves reflected by the compression unit 35. In addition, the image combining unit 77 positionally associates the compressed breast image with the mammographic image under the control of the control processor 78.

The control processor 78 has a function as an information processing apparatus (computer) and controls the overall operation of the ultrasonic diagnosis apparatus. The control processor 78 reads out control programs for image generation/display and the like from the internal storage unit 79, maps them in its internal memory, and executes computation/control and the like associated with various types of processing. The control processor 78 also implements an ultrasonic scan function of distributing scanning lines fanwise and the compressed breast image reconstruction function by mapping dedicated programs in the memory.

The internal storage unit 79 stores transmission/reception conditions, a control program for executing image generation/display processing, diagnosis information (a patient ID, findings by a doctor, and the like), a diagnosis protocol, the scanning line angle information of the probe, the position information of the probe, a body mark generation program, a dedicated program for implementing the compressed breast image reconstruction function, and other data. The internal storage unit 79 is also used to store images in the image memory 76 as needed. Data from the internal storage unit 79 can be transferred to another apparatus such as the mammographic diagnosis apparatus 2 via the interface unit 80.

The interface unit 80 is an interface associated with the input device 64, a network, and a new external storage device (not shown). The interface unit 80 can transfer data such as ultrasonic images and analysis results which are obtained by the apparatus to another apparatus via a network.

### (Compressed Breast Image Reconstruction Function)

The compressed breast image reconstruction function which the breast imaging system 1 has will be described next. This function generates a compressed breast image by mapping an echo signal obtained by ultrasonic scanning in correspondence with a compressed two-dimensional or three-dimensional breast shape on the basis of information about the propagation path of ultrasonic waves including the reflection of ultrasonic waves by the compression unit. For a concrete description, this embodiment exemplifies a case in which after a general ultrasonic image is generated on the basis of an echo signal, a compressed breast image is reconstructed by using the general ultrasonic image. The generation of a general ultrasonic image is not however essential. It suffices to generate a compressed breast image by directly mapping an echo signal or mapping values originating from the signal in correspondence with a compressed breast shape on the basis of information about the propagation path of ultrasonic waves including the reflection of ultrasonic waves by the compression unit.

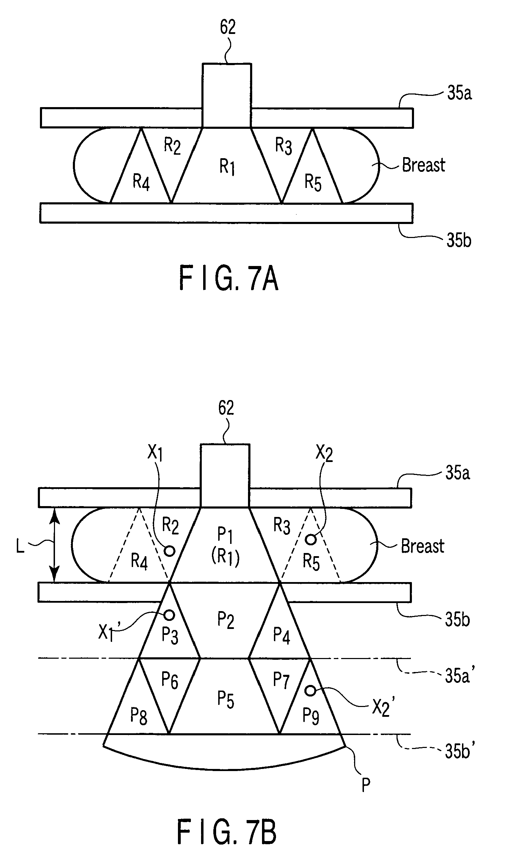

FIGS. 7A and 7B are views for explaining the compressed breast image reconstruction function. FIG. 7A shows that the breast compressed/fixed by the compression unit 35 is divided into real areas R1 to R5. FIG. 7B shows that a general ultrasonic image P obtained by mapping an echo signal, which is obtained by placing the ultrasonic probe 62 in the opening portion 350 and executing ultrasonic scanning, into a sector shape is divided into small image areas P1 to P9. Note that the small image areas P1 (R1), P2, and P5 are mirror images of each other, and so are the small image areas P3 and P6, and the small image areas P4 and P7. Although the small image area P1 corresponds to the real area R1, the remaining small image areas do not correspond to any real areas. In this sense, the small image areas P2 to P9 are virtual images, and the ultrasonic image P including them can be said to be a virtual image.

The small image area P1 in FIG. 7B is an image generated by using only reflected waves from tissue in the breast compressed/fixed by the compression unit 35, and corresponds to an image directly visualized from the real area R1. In addition, the value (i.e., the echo signal strength) at an arbitrary point X1' in the small image area P3 in FIG. 7B actually originates from an ultrasonic wave applied from the ultrasonic probe 62 and reflected along the path of the lower paddle 35b, the point X1 (in the real area R2) symmetrical to the point X' with respect to the lower paddle 35b, and the lower paddle 35b. Therefore, the real area R2 can be visualized by the small image area P3. Likewise, the real area R3 can be visualized by the small image area P4.

The value (i.e., the echo signal strength) at an arbitrary point X2' in the small image area P9 in FIG. 7B actually originates from an ultrasonic wave applied from the ultrasonic probe 62 and reflected along the path of the lower paddle 35b, the upper paddle 35a, a point X2 (in the real area R5) symmetrical to the point X2' with respect to the lower paddle 35b and an virtual image upper paddle 35a', the upper paddle 35a, and the lower paddle 35b. Therefore, the real area R5 can be visualized by the small image area P9. Likewise, the real area R4 can be visualized by the small image area P8. The real area R2 can be visualized from the small image area R6. The real area R3 can be visualized from the small image area R7.

As described above, the breast can be visualized in the form compressed/fixed by the compression unit 35 by reconstructing an image such that the real area R1 is replaced by the small image areas P1, P2, and P5; the real area R2, by the small image area P3 or P6; the real area R3, by the small image area P4 or P7; the real area R4, by the small image area P8; and the real area R5, by the small image area P9.

In this reconstruction, there is no specific limitation on which small image area is to replace each real area. For example, when the ultrasonic image P is divided into a plurality of layers in the depth direction with the distance between the upper and lower paddles 35a and 35b being represented by L, an image can be reconstructed by using small image areas including echo signals exhibiting two or three reflections of ultrasonic waves by the compression unit 35 in such a manner that the real areas R1, R2, R3, R4, and R5 are respectively replaced by the small image areas P5, P6, P7, P8, and P9. Performing image reconstruction upon selecting small image areas in accordance with the number of reflections by the compression unit 35 can reconstruct an image by using reflected waves exhibiting similar degrees of attenuation and improve image quality. In addition, for example, with regard to an area outside the real area R4 or R5, image reconstruction can be implemented by using a small image area below the virtual image lower paddle 35b'.

Note that the processing (compressed breast image reconstruction processing) based on the above compressed breast image reconstruction function requires information concerning the distance L between the upper and lower paddles 35a and 35b in addition to the ultrasonic image P obtained by ultrasonic scanning on the breast compressed/fixed by the compression unit 35. This information can be acquired from the mammographic diagnosis apparatus 2 by communication. However, the present invention is not limited to this. For example, such information can be obtained on the basis of the distance between the upper and lower paddles 35a and 35b visualized in the ultrasonic image P.

Such compressed breast image reconstruction processing is executed for each ultrasonic image obtained by volume scanning, thereby generating a plurality of compressed breast images corresponding to the respective ultrasonic images. It is possible to generate volume data concerning the compressed breast from the obtained compressed breast images. The user can select and observe an arbitrary slice image (a B mode image, C mode image, or the like) of the compressed breast by using the volume data concerning the compressed breast.

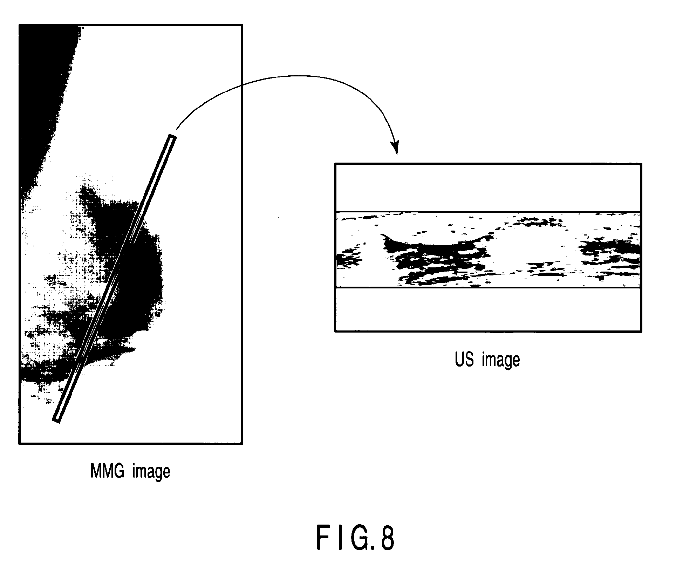

After the execution of the compressed breast image reconstruction processing, the central control unit 50 or the image combining unit 77 positionally associates the reconstructed compressed breast image with the mammographic image. This positional association can be implemented by providing a position sensor in the opening portion 350 of the compression unit 35 or the ultrasonic probe 62 itself and specifying the position of the ultrasonic probe 62 on the flat panel detector 40. At this time, as shown in FIG. 8, it is preferable to indicate the position of the ultrasonic image on the mammographic image (or the position of the mammographic image on the ultrasonic image) in advance and, when the operator clicks the position as needed, automatically display the ultrasonic image (or the mammographic image) corresponding to the position simultaneously with the mammographic image (or the ultrasonic image) or selectively.

### (Ultrasonic Scanning)

When executing the above compressed breast image reconstruction processing, this apparatus executes ultrasonic scanning so as to distribute scanning lines fanwise as shown in FIG. 4. This facilitates the occurrence of ultrasonic reflection in the compression unit 35 and allows wide-area ultrasonic imaging along the surface direction of the compression unit 35.

Note that such ultrasonic scanning can be implemented by calculating a delay time for phase control in accordance with the position of a scanning line and controlling the driving timing of each piezoelectric transducer on the basis of the calculated position.

### (Operation)

The operation of the breast imaging system 1 which includes compressed breast image reconstruction processing will be described next.

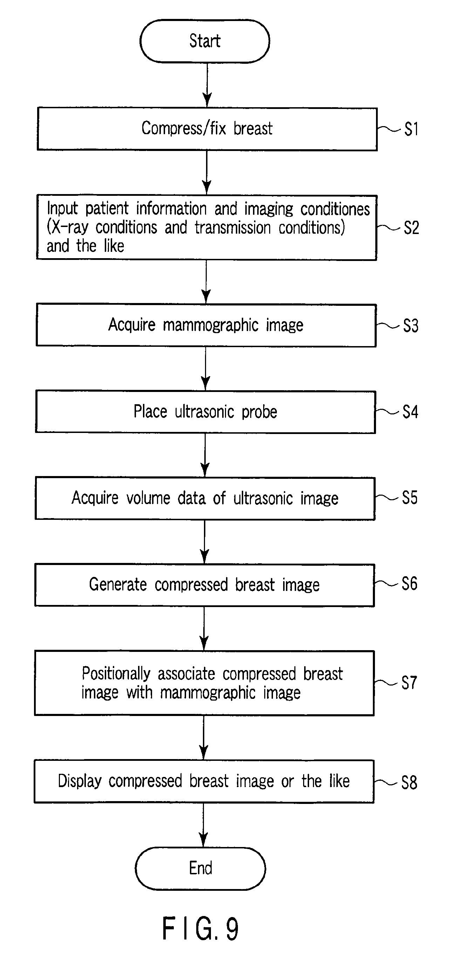

FIG. 9 is a flowchart showing each processing sequence executed in image acquisition using the breast imaging system 1. As shown in FIG. 9, first of all, a breast of a patient is compressed/fixed by the upper paddle 35a and the detection surface (i.e., the lower paddle 35b) of the flat panel detector 40 to position the subject (step S1). The operator then inputs patient information, imaging conditions, and the like to the mammographic diagnosis apparatus 2 and the ultrasonic diagnosis apparatus 6 via the operation unit 51 and the input device 64 (step S2). The imaging conditions in the case of the mammographic diagnosis apparatus 2 indicate X-ray conditions (a tube voltage, mAs value, focal point - imaging plane distance, delay time for each channel which implements a fan-like scanning line distribution, and the like). The imaging conditions in the case of the ultrasonic diagnosis apparatus 6 indicate transmission conditions (a transmission voltage, focal position, and the like). The central control unit 50 of the mammographic diagnosis apparatus 2 applies X-rays in accordance with the input imaging conditions, and acquires a mammographic image on the basis of the X-rays transmitted through the breast (step S3).

The ultrasonic probe 62 is then placed in the opening portion 350 of the upper paddle 35a (step S4). Volume data concerning the breast is acquired by performing ultrasonic scanning while the ultrasonic probe 62 is slid (step S5). The image combining unit 77 generates a compressed breast image by performing known reconstruction processing for each ultrasonic image constituting the acquired volume data (step S6).

The central control unit 50 of the mammographic diagnosis apparatus 2 or the control processor 78 of the ultrasonic diagnosis apparatus 6 positionally associates the mammographic image with the compressed breast image on the basis of the position of the ultrasonic probe 62 on the flat panel detector 40 (step S7). The display unit 47, the monitor 63, or another display device displays the mammographic image and the compressed breast image, which are associated with each other, in a predetermined form (step S8).

### (Effects)

According to the above arrangement, the following effects can be obtained.

This breast imaging system can perform ultrasonic scanning on a breast sufficiently compressed/fixed by compression paddles smaller in the number of ultrasonic probe insertion opening portions than in the prior art. Therefore, an ultrasonic image can be acquired with the same placement of the subject as that at the time of acquisition of a mammographic image, and a compressed breast image can be reconstructed by using the ultrasonic image. This makes it possible to acquire a mammographic image and a compressed breast image which can be easily associated with each other and compared with each other. In addition, this allows accurate and easy positional association between the images.

In addition, according to this breast imaging system, volume data concerning a compressed/fixed breast can be acquired by using the volume data obtained by fitting the ultrasonic probe in the opening portion and performing ultrasonic scanning while sliding the probe in a direction perpendicular to the ultrasonic scanning plane. This can shorten the time required for the acquisition of an ultrasonic image and reduce the burden on a patient as compared with the

### prior art.

In this breast imaging system, the opening portion of a compression paddle only needs to have a size large enough to fit the ultrasonic probe therein and slide it. Therefore, it is not necessary to compress a breast between the compression paddles having a plurality of holes. This can reduce pain in the breast when it is compressed/fixed, and can also prevent a decrease in compression strength.

### (Second Embodiment)

The second embodiment of the present invention will be described next. This embodiment exemplifies a breast imaging system 1 which implements a compressed breast image reconstruction function using a two-dimensional ultrasonic probe.

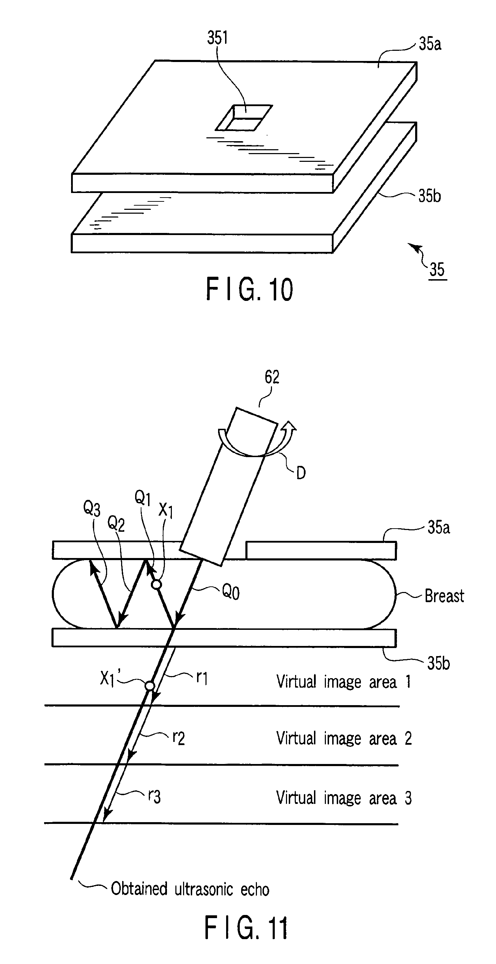

When a two-dimensional array probe having a two-dimensional array of ultrasonic transducers is to be used, volume data can be acquired by three-dimensional scanning without moving the probe along an opening portion 350. If an ultrasonic probe 62, therefore, is a two-dimensional array probe, a compression unit 35 has an opening portion 351 having a shape conforming to the two-dimensional array probe as shown in FIG. 10.

Note that an image combining unit 77 can generate an arbitrary compressed breast image by performing compressed breast image reconstruction processing for each arbitrary slice constituting acquired volume data. In addition, performing compressed breast image reconstruction processing for each slice constituting the acquired volume data makes it possible to acquire volume data concerning the compressed/fixed breast.

In addition, it is also possible to acquire volume data concerning a compressed/fixed breast by directly mapping echo signals obtained by ultrasonic scanning in correspondence with the compressed three-dimensional breast shape on the basis of information concerning the propagation paths of ultrasonic waves which includes the reflection of ultrasonic waves by the compression unit.

The above arrangement can further reduce pain in a breast when it is compressed/fixed and prevent a decrease in compression strength as compared with the first embodiment.

### (Third Embodiment: Trapezoid)

The third embodiment of the present invention will be described next. This embodiment exemplifies a breast imaging system 1 which implements a compressed breast image reconstruction function using a one-dimensional array ultrasonic probe.

When a one-dimensional array probe having a one-dimensional array of ultrasonic transducers is to be used, an ultrasonic probe 62 is placed in, for example, an opening portion 351 shown in FIG. 10, and ultrasonic transmission/reception is performed such that an ultrasonic scanning plane becomes a fan-like shape (e.g., a trapezoidal shape).

This arrangement can acquire an ultrasonic image of a desired slice of a breast without moving the probe 62 along the opening portion 350. This can further reduce pain in a breast at the time of image diagnosis.

### (Fourth Embodiment: Tilting)

The fourth embodiment of the present invention will be described next. This embodiment exemplifies a case in which volume data is acquired by using a one-dimensional array probe 62 without moving it along an opening portion 350.

FIG. 11 is a view for explaining a compressed breast image reconstruction function according to this embodiment. An ultrasonic wave transmitted to a scanning plane Q0 in a compressed breast sequentially propagates in the breast through, for example, paths Q1, Q2, and Q3 in the order named while being attenuated and reflected by the surfaces of the compression paddles shown in FIG. 11. Therefore, for example, the reflected wave from the propagation path Q1 differs in reception time (reception timing) from the reflected wave from the propagation path Q2. The positions on ultrasonic wave propagation paths in the breast from which reflected waves are received are discriminated on this difference in reception time, and the waves are converted and mapped as shown in FIG. 11, thereby generating a two-dimensional ultrasonic image with the propagation paths Q1, Q2, and Q3 serving as scanning planes.

That is, a reflected wave corresponding to an ultrasonic wave which is reflected once by a compression unit 35 and propagates through the propagation path Q1 (e.g., a reflected wave from an arbitrary point X1) is mapped at a position (point X1') corresponding to a virtual image area r1 having a mirror image relationship with the propagation path Q1 in accordance with the reception timing. Likewise, a reflected wave corresponding to an ultrasonic wave which is reflected twice by the compression unit 35 and propagates through the propagation path Q2 is mapped at a position corresponding to a virtual image area r2, and a reflected wave corresponding to an ultrasonic wave which is reflected three times by the compression unit 35 and propagates through the propagation path Q3 is mapped at a position corresponding to a virtual image area r3.

It is also possible to reconstruct volume data concerning a virtual image of the breast by performing similar mapping processing for each scanning plane with respect to the volume data of the breast which is obtained by tilting the scanning plane Q0 along a direction D using a swinging unit (not shown).

The above arrangement can acquire an ultrasonic image of a desired three-dimensional area in a breast without moving the probe 62 along the opening portion 350. This can therefore further reduce pain in a breast at the time of image diagnosis.

The present invention is not limited to the above embodiments, and constituent elements can be modified and embodied in the execution stage within the spirit and scope of the invention. The following are concrete modifications.
(1) Each function according to the embodiments can also be implemented by installing programs for executing the corresponding processing in a computer such as a workstation and unarchiving them in a memory. In this case, the programs which can cause the computer to execute the corresponding techniques can be distributed by being stored in recording media such as magnetic disks (floppy (registered trademark) disks, hard disks, and the like), optical disks (CD-ROMs, DVDs, and the like), and semiconductor memories.
(2) In this breast imaging system, the angle of an ultrasonic scanning line can be arbitrarily changed. Although there is no specific limitation on how the angle is changed, an arbitrary value or a preset value can be artificially selected on the basis of the size of a breast of a patient, the width and length of a compression paddle, a distance L between upper and lower paddles 35a and 35b, and the like.
(3) Each embodiment described above has exemplified the arrangement in which the ultrasonic probe is set in an X-ray path area. However, the present invention is not limited to this. It suffices to place an ultrasonic probe outside an X-ray path area (for example, between the upper and lower paddles 35a and 35b) and execute breast imaging by the same technique as in each embodiment.

In addition, various inventions can be made by proper combinations of a plurality of constituent elements disclosed in the above embodiments. For example, several constituent elements may be omitted from all the constituent elements disclosed in the above embodiments. Furthermore, constituent elements in different embodiments may be properly combined.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. A breast imaging system (1) for acquiring an X-ray image and ultrasonic image of a breast, the system (1) **characterized by** comprising:
an X-ray application unit (33) which applies X-rays;
an X-ray detection unit (40) which detects X-rays striking a detection surface;
a compression unit (35) which has an opening portion (350) in which an ultrasonic probe (62) is to be placed and compresses the breast by sandwiching the breast between a first paddle and a second paddle;
an X-ray image generating unit (43) which generates an X-ray image on the basis of X-rays detected by the X-ray detection unit (40);
an ultrasonic probe (62) which is placed in the opening portion (350), and generates an echo signal by transmitting an ultrasonic wave to the compressed breast on the basis of a supplied driving signal, and receiving a reflected wave from the breast;
a transmission unit (71) which supplies the driving signal to the ultrasonic probe (62); and
an image generating unit (75) which generates an ultrasonic image of the compressed breast on the basis of the echo signal and information concerning a propagation path of an ultrasonic wave including reflection of an ultrasonic wave by the compression unit (35).

2. A system (1) according to claim 1, **characterized in that**
the information concerning the propagation path is determined on the basis of an ultrasonic transmission/reception time for the compressed breast and a distance between the first compression paddle and the second compression paddle, and
the image generating unit (75) generates an ultrasonic image of the compressed breast by two-dimensionally mapping one of the echo signal and a value originating from the echo signal on the basis of the information concerning the propagation path.

3. A system (1) according to claim 1, which further comprises a calculation unit (78) which calculates a delay time for each scanning line so as to set a scanning line distribution of ultrasonic waves, transmitted to the compressed breast, fanwise, and **characterized in that**
the transmission unit (71) controls a supply timing of the driving signal on the basis of the calculated delay time.

4. A system (1) according to claim 1, **characterized in that** the reconstruction unit generates an ultrasonic image of the compressed breast by using an echo signal corresponding to one of ultrasonic waves reflected once and twice by the compression unit (35).

5. A system (1) according to claim 1, **characterized in that** the reconstruction unit generates an ultrasonic image of the compressed breast by using an echo signal corresponding to one of ultrasonic waves reflected twice and three times by the compression unit (35).

6. A system (1) according to claim 1, **characterized by** further comprising:
a positional association unit which associates a position of the X-ray image with a position of an ultrasonic image of the compressed breast; and
a display unit (47, 63) which displays the position of the associated ultrasonic image of the compressed breast on the X-ray image.

7. A system (1) according to claim 6, which further comprises a designation unit which designates a position of an ultrasonic image of the compressed breast on the X-ray image, and **characterized in that**
the display unit (47, 63) displays the ultrasonic image of the compressed breast when the position of the ultrasonic image of the compressed breast on the X-ray image is designated.

8. A system (1) according to claim 1, **characterized by** further comprising:
a positional association unit which associates a position of the X-ray image with a position of an ultrasonic image of the compressed breast; and
a display unit (47, 63) which displays the associated position of the X-ray image on the ultrasonic of the compressed breast.

9. A system (1) according to claim 8, which further comprises a designation unit which designates a position of the X-ray image on an ultrasonic image of the compressed breast, and **characterized in that**
the display unit (47, 63) displays the X-ray image when a position of the X-ray image on the ultrasonic of the compressed image is designated.

10. A system (1) according to claim 1, **characterized in that** the image reconstruction unit generates volume data concerning a breast fixed to the compression paddle by using a plurality of ultrasonic images of the compressed breast at different spatial positions.

11. A system (1) according to claim 1, **characterized in that** the opening portion (350) has a shape that allows an ultrasonic probe (62) to slide in a direction perpendicular to an ultrasonic scanning plane while the probe (62) is fitted in the opening portion (350).

12. A system (1) according to claim 1, **characterized in that** the compression paddle is made of a material having high ultrasonic reflectivity.

13. A system (1) according to claim 1, **characterized in that** the opening portion (350) of the compression paddle is provided with a thin film made of an acoustically transparent material.

14. A system (1) according to claim 1, **characterized by** further comprising a swinging unit which swings the ultrasonic probe (62) set in the opening portion (350).

15. An ultrasonic diagnosis apparatus (6) **characterized by** comprising:
an ultrasonic probe (62) which generates an echo signal by transmitting an ultrasonic wave to a breast compressed by the compression paddle on the basis of a supplied driving signal, and receiving a reflected wave from the breast;
a transmission unit (71) which supplies the driving signal to the ultrasonic probe (62); and
an image generating unit (75) which generates an ultrasonic image of the compressed breast on the basis of the echo signal and information concerning a propagation path of an ultrasonic wave including reflection of an ultrasonic wave by the compression unit (35).

16. An apparatus (6) according to claim 15, **characterized in that**
the information concerning the propagation path is determined on the basis of an ultrasonic transmission/reception time for the compressed breast and a distance between the first compression paddle and the second compression paddle, and
the image generating unit (75) generates an ultrasonic image of the compressed breast by two-dimensionally mapping one of the echo signal and a value originating from the echo signal on the basis of the information concerning the propagation path.

17. An apparatus (6) according to claim 15, which further comprises a calculation unit (78) which calculates a delay time for each scanning line so as to set a scanning line distribution of ultrasonic waves, transmitted to the compressed breast, fanwise, and **characterized in that**
the transmission unit (71) controls a supply timing of the driving signal on the basis of the calculated delay time.

18. An apparatus (6) according to claim 15, **characterized in that** the reconstruction unit generates an ultrasonic image of the compressed breast by using an echo signal corresponding to one of ultrasonic waves reflected once and twice by the compression unit (35).

19. An apparatus (6) according to claim 15, **characterized in that** the reconstruction unit generates an ultrasonic image of the compressed breast by using an echo signal corresponding to one of ultrasonic waves reflected twice and three times by the compression unit (35).

20. An apparatus (6) according to claim 15, **characterized in that** the image reconstruction unit generates volume data concerning a breast fixed to the compression paddle by using a plurality of ultrasonic images of the compressed breast at different spatial positions.

21. An apparatus (6) according to claim 15, **characterized by** further comprising a swinging unit which swings the ultrasonic probe (62) set in the opening portion (350).

22. A breast imaging method of acquiring an X-ray image and ultrasonic image of a breast, the method **characterized by** comprising:
applying X-rays to a breast compressed by a compression unit (35) having an opening portion (350) in which an ultrasonic probe (62) is to be placed, a first paddle, and a second paddle;
detecting X-rays transmitted through the breast;
generating an X-ray image on the basis of the detected X-rays;
placing the ultrasonic probe (62) in the opening portion (350) and transmitting an ultrasonic wave to the compressed breast on the basis of a supplied driving signal;
generating an echo signal by receiving a reflected wave from the breast; and
generating an ultrasonic image of the compressed breast on the basis of the echo signal and information concerning a propagation path of an ultrasonic wave including reflection of an ultrasonic wave by the compression unit (35).

23. A breast imaging method **characterized by** comprising:
transmitting an ultrasonic wave to a breast compressed by a compression unit (35) on the basis of a supplied driving signal;
generating an echo signal by receiving a reflected wave from the breast; and
generating an ultrasonic image of the compressed breast on the basis of the echo signal and information concerning a propagation path of an ultrasonic wave including reflection of an ultrasonic wave by the compression unit (35).
